# EUROPEAN PATENT APPLICATION

(11) **EP 4 181 080 A1**
(43) Date of publication of application: **17.05.2023**
(21) Application number: 21208378.6
(22) Date of filing: 16.11.2021
(51) Int. Cl.: G06V 10/82, G06V 40/20, G06V 20/52

(54) **MONITORING AN ENTITY IN A MEDICAL FACILITY**

(71) Applicant: Koninklijke Philips N.V., 5656 AG Eindhoven (NL)
(72) Inventor: BRESCH, Erik, Eindhoven (NL); BOUTS, Mark Jacobus Rosalie Joseph, Eindhoven (NL); ZUO, Fei, Eindhoven (NL); VAN DER HEIDE, Esther Marjan, Eindhoven (NL)
(74) Representative: Philips Intellectual Property & Standards

(57) **Abstract**

A computer implemented method for use in monitoring a first entity in a medical facility comprises: i) obtaining an image of the medical facility, ii) using a machine learning process to fit a first articulated model to the first entity in the image, wherein the first articulated model comprises keypoints corresponding to joints and affinity fields that indicate links between the keypoints. The method further comprises iii) determining a location or posture of the first entity in the medical facility from relative locations of fitted keypoints of the first articulated model in the image.

## Description

### FIELD OF THE INVENTION

The disclosure herein relates to monitoring an entity (e.g. person, clinician, piece of equipment) in a medical facility.

### BACKGROUND OF THE INVENTION

Workflows (otherwise known as clinical workflows) are used in medical facilities (hospitals, clinics, etc.) to ensure that appropriate actions are taken for each patient, in a standardized manner. This helps ensure best practice in medical facilities and compliance with clinical guidelines. Workflows often specify a particular set of tasks or checks (items in the workflow) that should be performed with respect to the patient. Workflows may be used at all stages of the patient's treatment. for example, there may be a workflow associated with admitting the patient to the medical facility; another workflow associated with triage of the patient; and subsequent workflows that are used dependent on the particular issues or treatment pathways identified for the patient.

Workflow management (e.g. recording when actions in a workflow have been performed) is a significant, yet important overhead in medical facilities. As such, automated analysis, optimization, and control of clinical workflows is an ongoing area of active research.

Aside from workflow management, there are other tasks in medical facilities that it is desirable to automate, for example, equipment and/or patient tracking.

The disclosure herein aims to address these problems and others.

### SUMMARY OF THE INVENTION

Various projects have aimed to automate different aspects of work-flow management. Previous work in this area has, for example, tracked patients, medical staff, and equipment in hospital settings using infra-red light sensor tags with a view to improving resource allocation and avoiding supply bottlenecks, e.g., in an emergency department. However, such data is often comparatively coarsely resolved in time and space, and the subsequent semantic understanding of the clinical processes is far from easy.

Another project proposes the use of in-hospital video (infra-red and/or depth) data, which offers much richer information; it allows capture of the presence, location, and activities of multiple people, e.g., medical care givers and patients, as well as the use of medical equipment in great spatial and temporal detail. The room set-up and devices, in combination with the information on the people in an image can give a complete view of the context. Video monitors directly capture events such as a nurse changing an infusion pump, a nurse working with a monitor, a patient being sat in a chair for a certain time, etc. However, a significant challenge is the automation of such video analysis by means of computer algorithms.

In particular, clinical environments often present cluttered and highly complex scenes in which conventional classic image processing techniques for object detection and tracking tend to struggle or fail entirely.

Artificial Intelligence (AI) technology and, in particular, deep learning (DL) methods for large neural networks provide an opportunity for real-time video analysis. For example, the You Only Look Once (YOLO) algorithm described in the paper by J. Redmon, S. Divvala, R. Girshick and A. Farhadi ("You Only Look Once: Unified, Real-Time Object Detection," 2016 IEEE Conference on Computer Vision and Pattern Recognition (CVPR), 2016, pp. 779-788, doi: 10.1109/CVPR.2016.91) enables real-time identification and tracking of objects in a video stream. YOLO produces bounding boxes identifying the location of desired objects, but has the disadvantage that it is difficult to infer further semantic meaning from the video feeds using YOLO alone.

Another deep neural network solution, namely the "OpenPose" algorithm (Hidalgo et al., "Single-Network Whole-Body Pose Estimation", 2019) is capable of detecting humans in image and video data. OpenPose confers more information than YOLO, as the outputs include the locations of keypoints which might include (depending on the precise model used) the location of the head, shoulders, hips, elbows of the people in the images.

It has been realized by the inventors herein that algorithms such as OpenPose might be advantageously applied in medical facilities to extract semantic information from a video feed, allowing deeper understanding of the events taking place in the hospital. As will be described in more detail below, such semantic information may thus be used to update clinical workflows in a reliable and automated manner.

Thus, according to a first aspect herein there is a method for use in monitoring a first entity in a medical facility, the method comprising: i) obtaining an image of the medical facility; ii) using a machine learning process to fit a first articulated model to the first entity in the image, wherein the first articulated model comprises keypoints corresponding to joints and affinity fields that indicate links between the keypoints; and iii) determining a location or posture of the first entity in the medical facility from relative locations of fitted keypoints of the first articulated model in the image.

According to a second aspect there is a computer program product comprising computer readable medium the computer readable medium having computer readable code embodied therein, the computer readable code being configured such that, on execution by a suitable computer or processor, the computer or processor is caused to perform the method of the first aspect.

According to a third aspect there is an apparatus for use in monitoring a first entity in a medical facility. The apparatus comprises: a memory comprising instruction data representing a set of instructions; and a processor configured to communicate with the memory and to execute the set of instructions. The set of instructions, when executed by the processor, cause the processor to: i) obtain an image of the medical facility; ii) use a machine learning process to fit a first articulated model to the first entity in the image, wherein the first articulated model comprises keypoints corresponding to joints and affinity fields that indicate links between the keypoints; and iii) determine a location or posture of the first entity in the medical facility from relative locations of fitted keypoints of the first articulated model in the image.

Thus, in embodiments herein, an entity in a hospital is modelled in an articulated manner, using an articulated model comprising keypoints and affinity fields. It has been recognized that the flexibility of articulated models is well suited to complex and often cluttered scenes in medical facilities. It has furthermore been recognized that many pieces of medical equipment (ventilators and the like) may advantageously be fit using articulated models. The relative positions between the fitted keypoints and affinity fields allow the location and/or posture of an entity to be better determined. This can be use in various scenarios, for example to provide a semantic understanding of the hospital video feed that may be linked to workflows, for workflow automation in a hospital.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 is an example apparatus for monitoring a medical facility according to some embodiments herein;
Fig. 2 is an example method for monitoring a medical facility according to some embodiments herein;
Fig. 3a shows an example articulated model of a bed;
Fig. 3b shows an example articulated model of a ventilator;
Fig. 3c shows an example where an articulated model of a person is used to detect seizures;
Fig. 4a illustrates an example image (a photograph represented as a line drawing), overlain with two articulated models that have been fit to a patient in the image and a monitor in the image;
Fig. 4b illustrates an interaction between the fitted articulated models of the patient and the monitor illustrated in Fig. 4a;
Fig. 5 illustrates an example image (a photograph represented as a line drawing) of a bed and a monitor, where a first articulated model is fit to the bed and a second articulated model is fit to the monitor;
Fig. 6 illustrates an example image (a photograph represented as a line drawing) of two patient beds side by side, each with a patient therein who is interacting with a respective clinician; and
Fig. 7 illustrates a method according to some embodiments herein.

### DETAILED DESCRIPTION OF EMBODIMENTS

As noted above it is an object of embodiments described herein to provide improved semantic understanding of video streams in a medical environment (hospital, clinic, doctor's surgery, dentists, etc), particularly but non-exclusively, for use in automated workflow analysis.

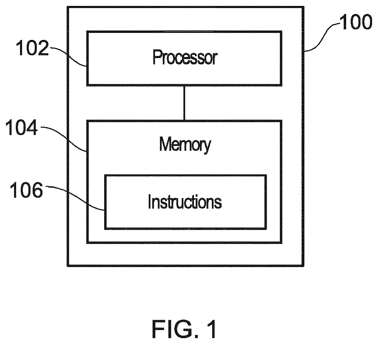

Turning now to Fig. 1 in some embodiments there is an apparatus 100 for use in monitoring a first entity in a medical facility, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions 106 and a processor 102 (e.g. processing circuitry or logic) configured to communicate with the memory and to execute the set of instructions. Generally, the set of instructions, when executed by the processor, may cause the processor to perform any of the embodiments of the method 200 as described below.

Embodiments of the apparatus 100 may be for use in monitoring a first entity in a medical facility. More specifically, the set of instructions, when executed by the processor, cause the processor to: i) obtain an image of the medical facility; ii) use a machine learning process to fit a first articulated model to the first entity in the image, wherein the first articulated model comprises keypoints corresponding to joints and affinity fields that indicate links between the keypoints; and iii) determine a location or posture of the first entity in the medical facility from relative locations of fitted keypoints of the first articulated model in the image.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the image, the first articulated model, the determined location or posture of the first entity and/or any other information or data received, calculated or determined by the processor 102 of the apparatus 100 or from any interfaces, memories or devices that are external to the apparatus 100. The processor 102 may be configured to control the memory 104 to store the image, the first articulated model, the determined location or posture of the first entity.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus may comprise an image acquisition unit with which to obtain the image in the medical facility. An image acquisition unit may comprise any audio-visual equipment capable of taking images or videos. E.g. a camera, infrared camera or similar. The image acquisition unit may be connected to the apparatus, for example, via a wired or wireless connection.

In another example, the apparatus may be configured to receive (e.g. via a wired or wireless connection) the image from an image acquisition unit that is separate to the apparatus 100.

In some examples, as described in more detail below, the apparatus 100 may further comprise a time-of flight (ToF) camera. A ToF camera produces an image matrix, where the value of each pixel is the distance/depth from the camera to the object. ToF cameras commonly use infrared imaging sensors. Such devices can produce both a "depth image" and a conventional infrared intensity image at the same time.

In other examples, the apparatus 100 may be configured to receive (e.g. via a wired or wireless connection) image(s) and or image matrix(s) from a ToF camera that is separate from the apparatus 100.

More generally, the apparatus 100 may further comprise a display. A display may comprise, for example, a computer screen, and/or a screen on a mobile phone or tablet, for displaying e.g. the image and/or the fitted model to a user. The apparatus may further comprise a user input device, such as a keyboard, mouse or other input device that enables a user to interact with the apparatus, for example, to provide initial input parameters (e.g. such as model selection) to be used in the method described herein. The apparatus 100 may comprise a battery or other power supply for powering the apparatus 100 or means for connecting the apparatus 100 to a mains power supply.

The apparatus may be used in a medical facility. Examples of a medical facility include, but are not limited to a hospital, clinic, doctor's surgery, dentist and veterinary clinic. As described above, a medical facility may use workflows (otherwise known as clinical workflows) to monitor activities taking place in the facility.

The apparatus is for monitoring a first entity in the medical facility. In this sense, the first entity may be any object, person or animal in the medical facility. For example, the first entity may be a person such as a patient, a caregiver, a doctor, a nurse, a surgeon, or a cleaner in the medical facility. As another example the first entity may be a piece of equipment in the medical facility, for example, such as a ventilator, a monitor, an SpO2 device or a vital sign monitor. As a further example still, the first entity may be any other inanimate object in the medical facility. For example, a hospital bed, chair, wheel-chair or walking apparatus.

As described above, the apparatus 100 comprises, or receives images from, an image acquisition unit. The image may be any image of the (e.g. inside of) a medical facility. The image may be a photographic image. The image may be in color (e.g. an RGB image) or in black and white. The image may be an infrared image or any other type of image modality.

As described below, the image may be a single frame, or be comprised in a video, e.g. as part of a sequence of video frames.

An image acquisition unit may form part of audio-visual equipment, such as a video camera. More than one camera/video camera may be implemented in the medical facility. Such cameras/video cameras may be placed so as to provide continuous coverage of part of the inside of the medical facility. As an example, the image or video stream may be obtained from a system akin to an integrated closed-circuit television (CCTV) system.

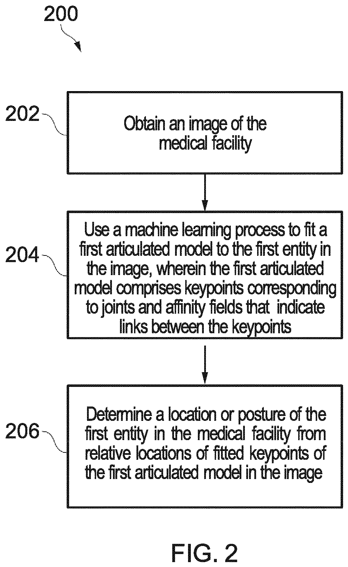

Turning to Fig. 2, there is a computer implemented method 200 for use in monitoring a first entity in a medical facility. Embodiments of the method 200 may be performed, for example by an apparatus such as the apparatus 100 described above.

Briefly, in a first step 202, the method 200 comprises: i) obtaining an image of the medical facility. In a second step 204 the method 200 comprises: ii) using a machine learning process to fit a first articulated model to the first entity in the image, wherein the first articulated model comprises keypoints corresponding to joints and affinity fields that indicate links between the keypoints. In a third step 206, the method comprises iii) determining a location or posture of the first entity in the medical facility from relative locations of fitted keypoints of the first articulated model in the image.

As described above, step 202 (comprising i) obtaining an image of the medical facility) may be performed in different ways. For example, the image may be received from an image acquisition unit (e.g. in real time, or near real-time). In other examples the image may be retrieved from a server, or database of images and videos, or similar.

The image may show part of the medical facility, for example, part of the inside or outside of a medical facility. The image may be an image of a ward, clinic, or assessment room, for example.

In step 204 the method 200 comprises ii) using a machine learning process to fit a first articulated model to the first entity in the image, wherein the first articulated model comprises keypoints corresponding to joints and affinity fields that indicate links between the keypoints.

In other words, an articulated model is fit to the first entity in the image. An articulated model, otherwise known as a "skeleton model" or articulated skeleton, comprises keypoints, corresponding to joints in the model. The keypoints may correspond to landmarks on the first entity. Generally, each keypoint corresponds to a point, or particular location on the first entity.

The keypoints may correspond to (literal) joints, such as flexible joints that allow for rotation or movement of the structures represented by the affinity fields either side of the keypoint. Joints may be e.g. pivot points on the first entity. For example, if the first entity is a person (e.g. patient, clinician etc), then one or more keypoints may be defined in the first articulated model corresponding to one or more anatomical joints of the person. For example, one or more keypoints may be defined corresponding to the hip joint, shoulder joint, knee, elbow, or any other joint on the body of the person. If the first entity is a piece of equipment, then one or more keypoints may be defined in the first articulated model that correspond to a join or joint in the equipment. For example, if the first entity is a ventilator, then a keypoint may be defined corresponding to the point where a mask is attached to a hose of the ventilator.

In addition to joints, the first articulated model may further comprise keypoints that correspond to landmarks on the first entity. For example, if the first entity is a person, the first articulated model may further comprise keypoints for other landmarks on the person, for example, particular anatomical features e.g. to the eyes, nose, or shoulders of the person. If the first entity is a piece of equipment, then the keypoints may correspond to particular landmarks on the piece of equipment, such as e.g. an outer extremity (e.g. edge or corner) of the equipment, or e.g. a mask.

The first articulated model may comprise one or more keypoints corresponding to joints and one or more keypoints corresponding to landmarks. E.g. a mixture of keypoint types. Generally, when designing an articulated model, keypoints should be chosen from salient characteristic image features of the object. E.g., the eyes of a person, or wheels of a hospital bed. Furthermore, for an articulated model, the keypoints should coincide with the joints or hinges of the object. It will be appreciated that these are merely examples and that keypoints may be defined for a great number of different locations on the first entity, depending on the nature of the first entity being modelled.

The first articulated model further comprises affinity fields. The affinity fields indicate or correspond to links between the keypoints.

One or more affinity fields in the first articulated model may correspond to physical links. For example, if the first entity is a person, examples of physical links include but are not limited to a thigh (which would be represented by an affinity field located between keypoints corresponding to the hip and knee), and a forearm (which would be represented by an affinity field located between keypoints corresponding to a hand and an elbow). If the first entity is a ventilator, then a physical link may correspond to a hose (located between two keypoints corresponding to a mask and a base unit).

One or more affinity may also comprise logical links. A logical link may comprise or represent a positional relationship between two keypoints (e.g. between the locations on the first entity to which the keypoints correspond), even if they are not directly joined together (e.g. by a single corresponding piece of equipment or anatomy). For example, in the example of the first entity being a person, there is a logical link between the chin of the person and their collar bone, because even though the chin and collar bone are not directly linked, there is a positional relationship between them. Thus, in an articulated model of a person, an affinity field may be defined between two keypoints corresponding to the chin and collar bones.

The first articulated model may comprise one or more affinity fields corresponding to physical links and one or more affinity fields corresponding to logical links. E.g. a mixture of affinity field types.

Step 204 may comprise obtaining the first articulated model. This may be obtained from a database of articulated models, or defined, e.g. by a human Engineer.

The first articulated model may be represented as a tuple of co-ordinates (e.g. in a normalized co-ordinate system) and a tuple of vectors between different pairs of co-ordinates in the tuple of co-ordinates, where each coordinate in the tuple of coordinates corresponds to a keypoint, as described above, and each vector corresponds to an affinity field as described above. This is merely an example however and the skilled person will appreciate that the first model may be represented in a different manner to that described above.

As an example, if the first entity is a person (e.g. patient, doctor, nurse etc.), then a suitable first articulated model is defined in the OpenPose paper by Hidalgo et al. (2019).

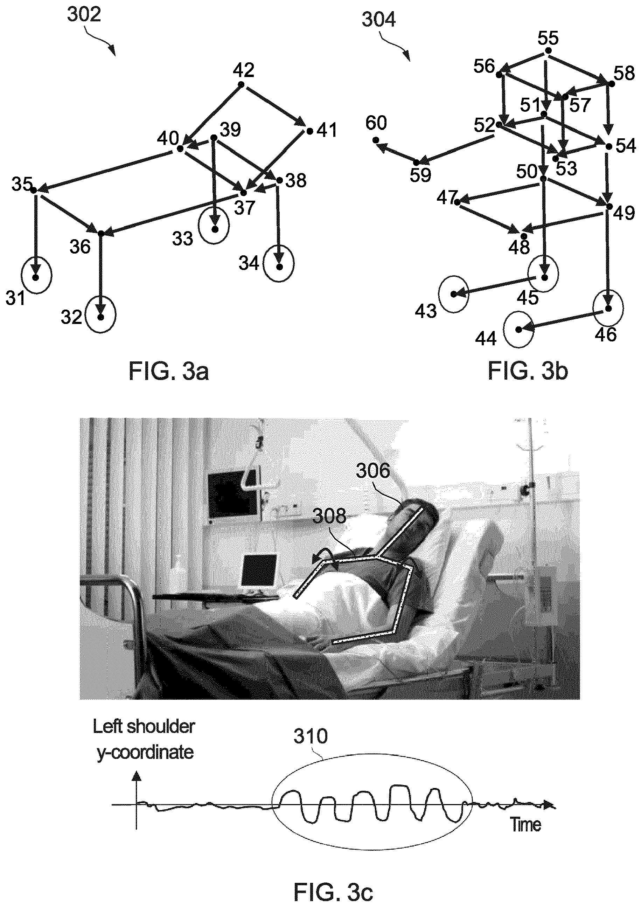

As another example, if the first entity is a bed 302 then the first articulated model may be defined as illustrated in Fig 3a. Fig. 3a shows an articulated model of a bed in the form of a directed graph. Its vertices are the keypoints, i.e., ["1-right front wheel", "2-left front wheel", ..., "12-right-top-headboard"]. The edges of the graph express the connectedness of the keypoints: [["35"→"31"], ["36"→"32"], ..., ["42"→"41"]].

As another example, if the first entity is a ventilator 304, then the first articulated model may be defined as illustrated in Fig 3b. In Fig 3b there is a ventilator (box with keypoints 51... 58), on a roll-stand (43... 46) with table (47... 50). The articulated ventilator tube is given by 52-59-60.

An advantage of modelling the entity as an articulated model (or skeleton) is that articulated models are inherently able to accommodate object-specific posture changes / deformations. For example, they can demarcate a human body's skeleton equally accurately no matter what the specific posture is, e.g., if an arm is raised or not. Similarly, an articulated model can be used to detect hospital beds no matter if the backrest is raised or not, because the backrest is an articulated component of the bed's articulated model.

The first articulated model is fit to the first entity in the image using a machine learning process. An example of a suitable machine learning process is the OpenPose method as described in Hidalgo et al. (2019). In Hidalgo, a first deep neural network is trained to transform image data into skeleton key-point heat-maps and part-affinity fields. A heatmap shows the likelihood that each pixel in the image is a keypoint. One heatmap is produced for each keypoint in the articulated model.

A part-affinity field is an image (consisting of two planes, one for the x-components, and one for the y-components), where each pixel corresponds to a 2-dimensional vector (an x-component, and a y-component). So, the part affinity field is a 2-dimensional vector field.

Further, a part-affinity field always belongs to a pair of keypoints, say "right elbow" and "right shoulder".

The (x,y)-vector at a particular pixel location in the part-affinity field encodes two pieces of information: (1.) The magnitude of the vector (x² + y²)^{½} indicates how likely the pixel belongs to a connection ("limb") between instances of the two keypoints (so, a "right upper arm" in our example). (2.) the direction of the (x,y)-vector at the pixel encodes the direction of the limb, i.e., where is the elbow and where is the shoulder.

This vector field information is used to determine, from the many candidates of keypoints (detected in the heatmaps), which pairs belong most likely together and form a limb. This is done by computing the path integral of the part-affinity vector field from the location of one keypoint candidate to another.

Note also, that this principle of encoding can be applied to more than 2 dimensions, e.g., 3 spatial dimensions in case of volumetric data, and/or the time dimension in case of, e.g., video data.

The heat-maps and part-affinity fields are then fed into a (bi-partite) graph matching stage (a.k.a., skeleton parser), which produces a skeleton-model based description of the object-content of the image. This may be performed using the method described in Hidalgo et al. (2019).

Thus, put another way, the machine learning process may be performed in two steps. Firstly, a first deep neural network may be used to determine a first set of locations in the image corresponding to the keypoints in the first articulated model. This is akin to the use of a neural network to identify landmarks in an image. For example, the first deep neural network may be trained on a corpus of training images to identify the keypoints in the image, using a supervised learning process. The first neural network may output heat-maps and/or affinity fields as described above.

Secondly, a first graph-fitting process (or "skeleton parser") may be used that takes as input the locations in the image corresponding to the keypoints and the affinity fields in the first model to fit the first articulated model to the first entity in the image.

The output of the machine learning process (e.g. the output of the graph-fitting process) may, for example, be a list of the coordinates in the image of the keypoint locations for each detected object in the image. The output can thus be a list of lists of 2D-locations. For example, if a person is modelled as person = [head, left shoulder, right shoulder]; and there are two people in the image, then the output may be in the following form:
[person1, person2] =
[[[headX, headY],[leftShoulderX, leftShoulderY], [rightShoulderX, rightShoulderY]], [[headX, headY],[leftShoulderX, leftShoulderY], [rightShoulderX, rightShoulderY]]]

If no instances of the object are detected then in this example, the output is an empty list.

It will be appreciated that this is merely an example and that the inputs and outputs of the first articulated model may be different to those described in the examples above.

Turning back to step 206, the method comprises: iii) determining a location or posture of the first entity in the medical facility from relative locations of fitted keypoints of the first articulated model in the image. Once the positions of the different keypoints have been obtained from the fitted model in the image, the positions of the keypoints can be used to determine a location and/or posture of the first entity in the image.

Postures may be determined from the fitted keypoints in various ways, for example, a sequence of if-then statements may be used to determine whether the locations of fitted keypoints are consistent with a particular posture. For example, if the first entity is a person, then it may be determined that the person has their arm raised (i.e. is in a posture with their arm raised) if the position of a keypoint corresponding to a hand is higher in the image than that of a keypoint corresponding to the shoulder to which it is attached. As another example, if the first entity is a bed, then the posture of the bed may be labelled as "reclined" or "upright" dependent on the angle made by keypoints corresponding to the head of the bed and the body or main portion of the bed.

In other embodiments, in step 206 another machine learning model might be trained and used to provide a posture label on the basis of fitted keypoints. For example, a convolutional neural network may be trained to take as input the fitted keypoints and/or the image and output a label describing the posture. Training may be performed in a supervised manner using a training data set comprising examples of fitted keypoint locations and corresponding ground truth posture labels.

The determined location and/or posture of the first entity may be used to perform various tasks. Some examples are as follows:
Tracking of equipment. Often carts, (EMR) computers are scattered over departments and finding them takes substantial time. With this approach particular carts can be identified and located in an automated manner.
Object position checking. Certain patients may need specific body positions to guarantee safety and quick recovery (e.g. uplifted leg). As an example, an articulated model may be fit to a bed, and the angle of the bed may be determined from the fitted keypoints to confirm that the patient is in an upright position. If it is out of a predefined range, a signal might be provided that it is not in the specified range.

As another example, an articulated model may be fit to the patient, and it may be determined whether the patient's leg is raised using the fitted keypoints. This may be implemented, for example, as illustrated in the following pseudocode:
For each image in video stream do:
   Detect human(-skeleton) in image
If ankle/knee/hip are visible parts of detected skeleton:
   Compute location of patient's leg (using location coordinates & view point of installed camera)
If location is outside of permissible range:
   Raise alarm to caregiver: wrong patient position!
   Else:
   Raise warning to caregiver: patient position monitoring not possible

If a particular posture or patient location is required as part of a medical workflow, then this may be used as a trigger to perform the method 200 for that particular patient and particular location/posture. This may be used to automate this aspect of the workflow.

Turning now to other embodiments, as noted above, in some embodiments the image is a frame in a video and the method may further comprise repeating steps i), ii) and iii) on a sequence of frames in the video and determining a change in posture or a change in location of the first entity across the sequence of frames. Thus, the method 200 can be repeated in order to process video data and to monitor entities in the medical facility over time.

The analysis of video data may be used to determine changes in the location or posture of the first entity over time. For example, a change in location may be determined by comparing the locations of fitted keypoints in the first articulated model from one frame in the video to another. Similarly, a change in posture may be determined by determining the posture in a first image in the video, determining the posture in a second image in the video and determining the change in posture as the difference between the posture in the first image and that in the second image.

In some embodiments, the location or posture (or a change in location or posture) is used to determine whether an event has occurred with respect to the first entity. For example, where the first entity is a piece of medical equipment, a change in location or posture of the medical equipment may be used to determine whether the piece of medical equipment has been moved from a first location to a second location. As another example, where the first entity is a person, the event may comprise: the person exiting a bed, the person having a seizure, or the person remaining in one position for longer than a predefined time threshold.

For example, seizure detection may be performed tracking the location of body parts over time and raising an alarm when, e.g., large scale oscillations occur.

This is illustrated in Fig. 3c which shows a person 306 and a fitted articulated model 308 that has been fit to the person 306. The positions of keypoints may be plotted with respect to time. In the event of a seizure, oscillations indicted by the circle 310 in the positions of the keypoints may be detected as illustrated in the graph of the left-shoulder position with respect to time.

Detection of a medical condition such as a seizure may be used to trigger an automated update the patient's medical records (e.g. with details such as the time, date, location and/or duration of the seizure). Detection of a medical condition (such as a seizure) may further trigger a new workflow to be implemented for the patient. Furthermore, facial recognition may be used to ensure the correct medical record is updated. In this way, the method 200 can be used in automated record keeping and workflow management.

Turning now to other embodiments, the method 200 may further comprise fitting different articulated models to different entities in the image or images in a video and determining an interaction between different entities from the relative locations of their respective fitted keypoints. For example, steps i)-iii) above may be repeated for each entity in an image or sequence of images.

Put another way, the method 200 may further comprise using the machine learning process to fit a second articulated model to a second entity in the image. The second articulated model may comprise (a second set of) keypoints and (a second set of) affinity fields indicating links between the (second set of) keypoints. The method may then comprise determining an interaction between the first entity and the second entity in the image from relative locations of fitted keypoints of the first articulated model and fitted keypoints of the second articulated model. It will be appreciated that the method may further be extended to third and/or subsequent entities in an image.

Articulated models were described above with respect to the first articulated model and the details therein will be understood to apply equally to the second articulated model. For example, the second articulated model also comprises keypoints corresponding to joints and affinity fields that indicate links between the keypoints.

The first and second articulated models may be the same type of model, or different types of model, depending on the type of interaction that is being monitored for. For example, in an interaction between a patient and a doctor, the first and second articulated models will both be articulated models of humans.

The extension of the method 200 to first and second entities may be achieved in different ways. For example, if OpenPose (as described in Hidalgo et al. (2019)), is used in step 204, then what is proposed here, requires the extension of the network stage to include the detection of (new) key-point types, which are specific to the entity or entities that have to be detected. Naturally, along with the extension of the neural network architecture itself, also the training data set must be extended so that it includes appropriate instantiations of objects and a sufficient coverage of the naturally occurring variety of poses.

Furthermore, the skeleton parser has to be created so, that it parses the specific (new) object skeleton-models from the heat-maps and part-affinity fields, i.e., it requires an adjusted model description. Different options exist to accomplish this:
1) Using multiple, specially made OpenPose systems, for the different types of skeleton models, which are used in parallel. E.g., multiple different specialized systems being fed with the same video data. Put another way, a second deep neural network (that is different to the first deep neural network) may be trained and used to determine a second set of locations in the image corresponding to the keypoints in the second articulated model and a second graph-fitting process may be used that takes as input the locations in the image corresponding to the keypoints and the affinity fields in the second model to fit the second articulated model to the second entity in the image.
2) The multiple OpenPose systems can share the neural network processing trunk, and only employ different skeleton-model parsers. Put another way, the first deep neural network may be further trained and used to determine a second set of locations in the image corresponding to the keypoints in the second articulated model (e.g the same deep neural network may be used to determine the locations of the keypoints in the first articulated model in the image and the keypoints of the second articulated model in the image). A second (e.g different) graph-fitting process may then be used for the second entity, that takes as input the locations in the image corresponding to the keypoints and the affinity fields in the second model to fit the second articulated model to the second entity in the image.

Determining an interaction between the first and second entities may be performed, e.g. by detecting overlap between the fitted keypoints of the first articulated model and the fitted keypoints of the second articulated model.

In some embodiments, depth information may be used to improve the understanding of interactions between first and second entities in an image. And for example, to confirm that an interaction is a real interaction rather than merely a coincidental overlap between two entities in an image (e.g. due to camera angle). If depth images are used to locate the skeletons of objects/persons, the 3D coordinates of the objects/persons can be derived. Overtime the 3D coordinates form trajectory flows of the objects/persons. This gives more insights on the semantics of the scene. For example, if the hand/arm skeleton of the patient is lifted up which might indicate that the patient is trying to call for help or get attention. The depth information is also beneficial to resolve ambiguities and differentiate between occlusion and interaction.

Thus, the method 200 may further comprise determining depth information associated with fitted keypoints in the first articulated model and fitted keypoints in the second articulated model. The step of determining an interaction between the first entity and the second entity in the image may then be further based on the depth information.

Depth information may be determined, for example, using a time-of flight (ToF) camera. A ToF camera produces an image matrix, where the value of each pixel is the distance/depth from the camera to the object. ToF cameras commonly use particular infrared imaging sensors, and thus such a device could produce both the depth image and a conventional infrared intensity image at the same time.

Generally, depth information may be matched to fitted keypoints according to the following pseudocode:
For each image in video stream do:
Detect all objects(-skeletons) in 2D image (e.g., infrared or RGB image)
Lookup detected skeletons' keypoints in the depth image
From 2D & depth information compose 3D data location data of keypoints of the skeletons
Do further processing with 3D skeleton data (is more robust than 2D)

As an example of the "further processing" step in the example above, an interaction between a first entity (bed) and a second entity (patient) can be used to determine whether the patient has exited the bed. Bed exits may be determined if the 2D or 3D distance between the keypoints of the first articulated model (to the patient) and the fitted keypoints of the second articulated model (to the bed) exceeds a predefined threshold distance. This may be achieved, for example, according to the following pseudocode:
For each image in video stream do:
Detect human(-skeleton) in image
Detect bed(-skeleton) in image
If 3D distance (skeleton-to-bed) > threshold
Raise alarm to caregiver: patient exited the bed!

As another example, depth information may be used to determine that a ventilator is connected to a patient by means of an (articulated) tube, rather than, e.g., that merely a ventilator is present in the room, as would be the result of conventional object detection in photos/videos.

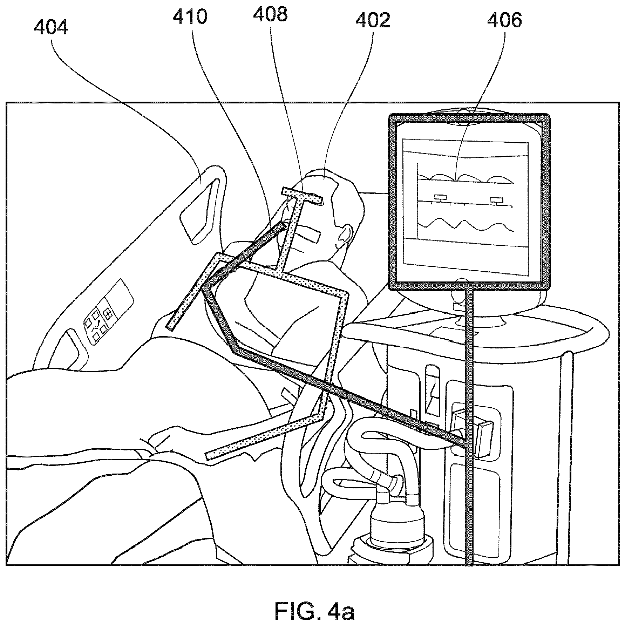

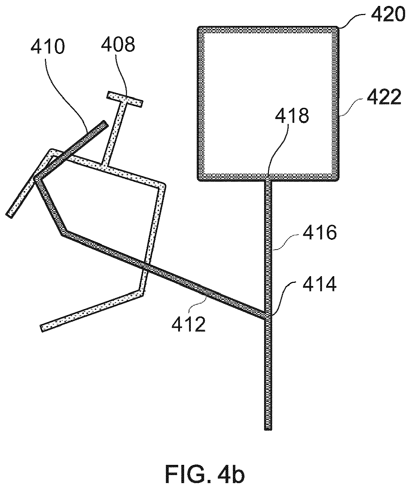

This is illustrated in Fig. 4a which shows a line drawing of an image in of a medical facility. The line drawing represents a photograph of the medical facility. It will be appreciated that in real life, the image would be a photograph, and may e.g. be in color. The image shows a patient 402 in a bed 404. The patient 402 is ventilated by means of a ventilator 406. A first articulated model 408 of a person has been fit to the patient 402. A second articulated model 410 of a ventilator has been fit to the ventilator 406. Fig. 4b shows the same fitted articulated models 408 and 410 as illustrated in Fig. 4a. In Fig 4b, a ventilation tube of the ventilator is represented by affinity field 412 and a stand associated with the ventilator is represented by affinity field 416. The joint between the two is represented by keypoint 414. Other keypoints on the ventilator include, for example, point 418 which represents the point between the stand 416 and a screen (the square structure on top of the stand). Corners of the screen are represented by keypoints such as 420 and edges by affinity fields (e.g. 422). In this example, the overlap between the skeletons 408 and 410 can be used to determine that the ventilator 406 is connected to the patient 402. If 3D coordinates are obtained (e.g. via a ToF camera) then these can be used to confirm that the patient is connected to the ventilator and rule out e.g. coincidental overlap.

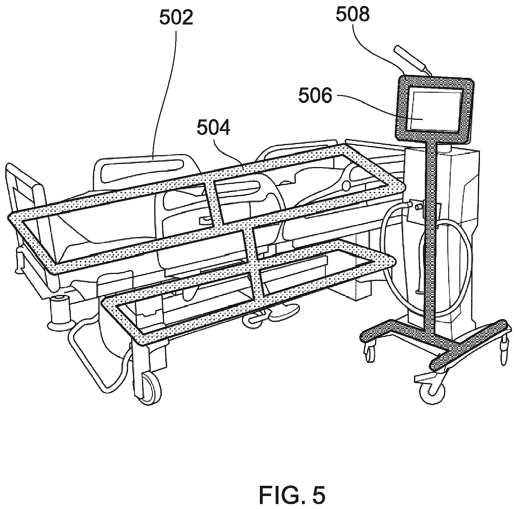

Fig. 5 shows another line drawing of an image in of a medical facility. The line drawing represents a photograph of the medical facility. It will be appreciated that in real life, the image may be a photograph, and may e.g. be in color. This example shows articulated models 504 and 508 fit to a bed 502 and a piece of medical equipment 506 respectively.

Generally, the interaction between the fitted articulated models of people and objects can provide insights that can be used to update clinical workflows. For example, the crossing/overlap of two skeletons can be interpreted as an interaction happening. As examples, the method 200 can be used to register events or data such as the frequency with which a nurse (first entity) operates a piece of equipment (second entity) such as a mobile patient monitor; if a nurse (first entity) interacts with an infusion pump (second entity), or whether a patient's bed is adjusted to seated or sleeping position.

In embodiments where the first entity is a piece of medical equipment and the second entity is a patient, the method 200 may be used to determine that an event has taken place between the piece of equipment and the patient. Examples include but are not limited to detection of the piece of equipment being attached to a patient, or detection of the piece of equipment being used to perform a medical procedure on a patient. Again, this information may be used to update one or more workflows or medical records associated with the patient.

In embodiments, where the first entity is a clinician and the second entity is a patient, the method 200 may be used to determine a first interaction between the clinician and the patient. Examples include but are not limited to the first interaction being: contact between the clinician and the patient; or a medical procedure being performed on the patient by the clinician. Facial recognition may further be used to link an interaction between a patient and a medical professional, to the correct patient and medical professional.

The following pseudo-code shows an example where interactions between each patient (first entity), clinician (second entity) and/or piece of medical equipment (third entity) as described above, are counted and used to provide statistics for use in workflow efficiency analytics and optimization:
For each image in video stream do:
   Detect all objects(-skeletons) in image (e.g., medical equipment, beds etc.)
   Detect all humans(-skeletons) in image
Optional: Classify humans into "nurse", "patient", "visitor" using
   (1) markers attached to humans' clothing (e.g., QR codes)
   (2) face recognition of each detected human's region in the image
Compute geometrical distance of humans to detected objects (e.g., human in bed => patient, human near medical equipment => nurse)
Count interactions between humans and objects (e.g., nurse interacting with patient, or bed, or medical equipment)
Compute frequency statistics of interactions and use in workflow efficiency analytics and optimization.

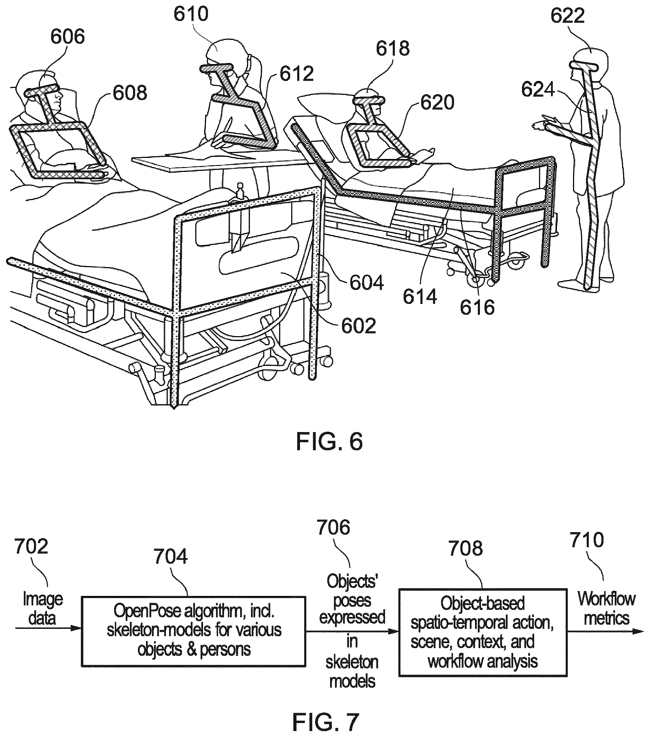

An output of this process is illustrated in Fig 6 which shows a line drawing of an image in of a medical facility. The line drawing represents a photograph of the medical facility. It will be appreciated that in real life, the image would be a pixelated photograph, and may e.g. be in color. The image shows a first bed 602, and a first patient 606 therein who is interacting with a first person 610. The image further shows a second bed 616 with a second patient 618 therein. A second person 622 is interacting with the second patient 618. In this example, the result of step: "Detect all objects(-skeletons) in image (e.g., medical equipment, beds etc.)" is that articulated model 604 is fit to bed 602, and articulated model 616 is fitted to bed 614. The result of step: "Detect all humans(-skeletons) in image" is that articulated model 608 is fit to first patient 606; articulated model 612 is fit to first person 610; articulated model 620 is fit to second patient 618 and articulated model 625 is fit to second person 622.

Turning now to Fig. 7, which illustrates a flow chart of a method for determining workflow metrics in a medical facility, using the method 200. This method may be performed by an apparatus such as the apparatus 100 described above. In this example, in step 702, an image (such as that illustrated in Fig. 6) is obtained according to step 202 of the method 200 described above. The image 702 is then fed into a machine learning process 704 that is used to fit one or more articulated models to one or more entities (e.g. patient, monitoring equipment) in the image. In this embodiment, the OpenPose machine learning process is used, as described above with respect to step 204 of the method 200. In this embodiment, the OpenPose method is extended with new articulated models corresponding to hospital equipment, beds and machines (as described above). In step 706, locations and/or postures of the one or more entities in the medical facility are determined from relative locations of fitted keypoints of the first articulated model in the image (as was described above with respect to step 206, above). In step 708, the locations and poses are then analyzed according to the methods described above, to determine actions, events and scene context. In step 710, metrics (e.g. such as contact metrics between patient and carer, or duration sat in a constant position etc.) are derived and these are used to update items in a workflow for the patient.

Turning now to other embodiments, as described above, the method 200 may be used to update and manage workflows. More generally, the method 200 may further comprise the location or posture of the first entity being used to determine whether an item in a clinical workflow has been performed, and updating the workflow with the result of the determination. For example, a location, posture or event pertaining to the first entity (patient), or an interaction between the first entity (patient) and a second entity (medical professional, medical equipment or other object in the medical facility) may be matched to an item in the workflow and/or trigger an update to said item.

As described above, in other embodiments, if a medical event or condition is detected for the patient (e.g. such as seizure detection, fall detection etc) using the method 200, then this may trigger the medical record of the patient to be updated, e.g. with data pertaining to the detected event or condition.

In other examples, an item in a clinical workflow may trigger performance of the method 200. For example, as noted above, if an item in the clinical workflow says that the patient must be in a particular posture (leg raised, reclined, upright etc) then this may trigger performance of the method 200 to determine whether the patient is in the particular posture.

In a similar manner, if an item in a clinical workflow says that a medical procedure should be performed on the patient, the method 200 may be used to identify whether an interaction between the clinician (first entity) and patient (second entity) is consistent with the medical procedure having been performed. The clinical workflow may thus be updated accordingly.

Turning now to other embodiments, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method for use in monitoring a first entity in a medical facility, the method comprising:
obtaining an image of the medical facility;
using a machine learning process to fit a first articulated model to the first entity in the image, wherein the first articulated model comprises keypoints corresponding to joints and affinity fields that indicate links between the keypoints; and
determining a location or posture of the first entity in the medical facility from relative locations of fitted keypoints of the first articulated model in the image.

2. A method as in claim 1 wherein the image is a frame in a video and wherein the method further comprises repeating steps i), ii) and iii) on a sequence of frames in the video; and
determining a change in posture or a change in location of the first entity across the sequence of frames.

3. A method as in claim 1 or 2 wherein the location or posture is used to determine whether an event has occurred with respect to the first entity.

4. A method as in claim 3 wherein:
the first entity is a person and wherein the event is:
the person exiting a bed;
the person having a seizure; or
the person remaining in one position for longer than a predefined time threshold; or wherein:
the first entity is a piece of medical equipment and wherein the event is:
the piece of medical equipment being moved from a first location to a second location;
the piece of equipment being attached to a patient; or
the piece of equipment being used to perform a medical procedure on a patient.

5. A method as in any one of the preceding claims further comprising:
using the machine learning process to fit a second articulated model to a second entity in the image, wherein the second articulated model comprises keypoints corresponding to joints and affinity fields that indicate links between the keypoints; and
determining an interaction between the first entity and the second entity in the image from relative locations of fitted keypoints of the first articulated model and fitted keypoints of the second articulated model.

6. A method as in claim 5 further comprising:
determining depth information associated with fitted keypoints in the first articulated model and fitted keypoints in the second articulated model; and
wherein the step of determining an interaction between the first entity and the second entity in the image is further based on the depth information.

7. A method as in claim 5 or 6 wherein the first entity is a clinician, the second entity is a patient and the first interaction is:
contact between the clinician and the patient; or
a medical procedure being performed on the patient by the clinician.

8. A method as in any one of the preceding claims wherein the step of using a machine learning process to fit a first articulated model to a first entity in the image comprises:
using a first deep neural network to determine a first set of locations in the image corresponding to the keypoints in the first articulated model; and
using a first graph-fitting process that takes as input the locations in the image corresponding to the keypoints and the affinity fields in the first model to fit the first articulated model to the first entity in the image.

9. A method as in claim 8 when dependent on claim 5, 6 or 7 further comprising:
using the first deep neural network to determine a second set of locations in the image corresponding to the keypoints in the second articulated model; and
using a second graph-fitting process that takes as input the locations in the image corresponding to the keypoints and the affinity fields in the second model to fit the second articulated model to the second entity in the image.

10. A method as in claim 8 when dependent on claim 5, 6 or 7 further comprising:
using a second deep neural network to determine a second set of locations in the image corresponding to the keypoints in the second articulated model; and
using a second graph-fitting process that takes as input the locations in the image corresponding to the keypoints and the affinity fields in the second model to fit the second articulated model to the second entity in the image.

11. A method as in any one of the preceding claims wherein the location or posture of the first entity is used to determine whether an item in a clinical workflow has been performed; and
updating the workflow with the result of the determination.

12. A method as in any one of claims 1 to 11 wherein the method is triggered by an item in a clinical workflow and wherein the location or posture of the first entity is used to determine whether the item has been performed; and
updating the workflow with the result of the determination.

13. A computer program product comprising computer readable medium the computer readable medium having computer readable code embodied therein, the computer readable code being configured such that, on execution by a suitable computer or processor, the computer or processor is caused to perform the method as claimed in any one of the preceding claims.

14. An apparatus for use in monitoring a first entity in a medical facility, the apparatus comprising:
a memory comprising instruction data representing a set of instructions; and
a processor configured to communicate with the memory and to execute the set of instructions, wherein the set of instructions, when executed by the processor, cause the processor to:
obtain an image of the medical facility;
use a machine learning process to fit a first articulated model to the first entity in the image, wherein the first articulated model comprises keypoints corresponding to joints and affinity fields that indicate links between the keypoints; and
determine a location or posture of the first entity in the medical facility from relative locations of fitted keypoints of the first articulated model in the image.

15. An apparatus as in claim 14 further comprising:
an image acquisition unit for obtaining the image; and/or
a time of flight camera to obtain image depth information for the fitted keypoints of the entity in the image.
